# EUROPEAN PATENT SPECIFICATION

(11) **EP 2 535 069 B1**
(45) Date of publication and mention of the grant of the patent: **18.12.2013**
(21) Application number: 11170015.9
(22) Date of filing: 15.06.2011
(51) Int. Cl.: A61M 1/16, A61M 1/34

(54) **Apparatus**
Vorrichtung
Dispositif

(43) Date of publication of application: 19.12.2012
(73) Proprietor: Gambro Lundia AB, 220 10 Lund (SE)
(72) Inventor: Neubauer, Markus, 82377 Penzberg (DE); Dietrich, Ruth, 72379 Hechingen (DE); Koch, Sylvia, 72458 Albstadt-Ebingen (DE); Hutzel, Kathrin, 72810 Gomaringen (DE)
(74) Representative: Perchenek, Nils

(56) References cited:
- EP-A1- 2 113 298
- WO-A1-96/31273
- JP-A- 2006 288 942
- KAWADA T ET AL: "Vagal stimulation suppresses ischemia-induced myocardial interstitial myoglobin release", LIFE SCIENCES, PERGAMON PRESS, OXFORD, GB, vol. 83, no. 13-14, 26 September 2008 (2008-09-26), pages 490-495, XP025433380, ISSN: 0024-3205, DOI: 10.1016/J.LFS.2008.07.013 [retrieved on 2008-07-31]
- LIANG H ET AL: "Determination of albumin and myoglobin in dialysate and ultrafiltrate samples by high-performance size-exclusion chromatography", JOURNAL OF CHROMATOGRAPHY B : BIOMEDICAL APPLICATIONS, ELSEVIER SCIENCE PUBLISHERS, NL, vol. 754, no. 1, 15 April 2001 (2001-04-15), pages 141-151, XP004232000, ISSN: 0378-4347, DOI: 10.1016/S0378-4347(00)00600-9

## Description

### Technical Field

The present disclosure relates to an apparatus for separation of myoglobin from blood by hemodialysis. The apparatus can be used in the therapy of rhabdomyolysis, which goes along with the presence of elevated levels of myoglobin in the blood serum.

### Description of the Related Art

The destruction of skeletal muscle tissue (rhabdomyolysis) leads to the release of the breakdown products of damaged muscle cells into the bloodstream; some of these breakdown products, such as myoglobin, are harmful to the kidney and may lead to acute kidney failure.

Successful removal of serum myoglobin using high cut-off hemodialysis (HCO-HD) has been reported (Crit Care 9 (2005) R90-R95; N Engl J Med 361 (2009) 1411-1413). The dialyzers used comprise membranes which *in vivo* allow passage of molecules having molecular weights of up to 45 kDa.

Successful treatment of rhabdomyolysis requires extensive removal of myoglobin from the serum of the patient. However, the time required to achieve a sufficient degree of removal is difficult to predict, as the treatment is not comparable to regular hemodialysis treatment, for which session parameters are well established.

Related devices are described in EP 2 113 298 A1 and JP 2006 288942 A. It would therefore be desirable to have a device which is able to automatically determine the optimal length of the treatment session, or to automatically regulate treatment parameters.

### Summary

The present invention provides an apparatus comprising a dialyzer capable of removing myoglobin from the serum of a patient. The device also measures the myoglobin level in the dialysate or filtrate, respectively, leaving the dialyzer during treatment, allowing the optimal length of the treatment session to be determined.

### Detailed Description

In one aspect of the invention, the apparatus comprises
i) a dialyzer comprising a blood compartment and a dialysate compartment, said compartments being separated from each other by a membrane which *in vivo* allows passage of myoglobin; and
ii) a device configured for on-line measurement of the myoglobin level in the dialysate leaving the dialyzer.

Dialyzers comprising a blood compartment and a dialysate compartment separated from each other by a membrane are known in the art. Examples of such dialyzers are plate dialyzers and capillary dialyzers. In plate dialyzers, the separating membrane takes the form of a sheet membrane or a stack of sheet membranes. Capillary dialyzers comprise a multitude of hollow fiber membranes.

An example of a capillary dialyzer suitable for use in the apparatus of the present invention comprises a housing defining a longitudinally extending internal chamber including a first end and a second end; a bundle of semipermeable hollow fiber membranes disposed within the internal chamber, the hollow fiber membranes extending longitudinally from the first end of the housing to the second end of the housing, the hollow fiber membranes having an outer surface, a first end and a second end corresponding to the first end and the second end of the internal chamber; end wall means supporting the first and second ends of the hollow fiber membranes within the internal chamber so as to sealingly separate the first and second ends of the hollow fiber membranes from the outer surface of the hollow fiber membranes between the first and second ends thereof; a first inlet for the introduction of a fluid into the first end of the housing, the first inlet being defined by a first end cap covering the first end of the housing; a first outlet for the evacuation of a fluid from the second end of the housing, the first outlet being defined by a second end cap covering the second end of the housing, the first and second end caps being applied to the first and second ends of the housing in a fluid-tight manner; a second inlet for the introduction of a fluid into the internal chamber at a location between the first and second end of the housing; and a second outlet for the evacuation of a fluid from the internal chamber at a location between the first and second end of the housing.

Dialyzers suitable for the apparatus of the present application comprise a membrane which *in vivo* allows passage of myoglobin. Such membranes show a sieving coefficient for myoglobin, determined in accordance with DIN EN 1283, of more than 0.1. In one embodiment, the sieving coefficient is larger than 0.5, for instance, 0.8 or higher, 0.9 or higher, or even 0.95 or higher. In order to avoid extensive loss of albumin, the sieving coefficient for albumin determined in accordance with DIN EN 1283, of suitable membranes is not more than 0.2, for instance, 0.15 or lower, or even 0.1 or lower. Dialyzers suitable for use in the apparatus of the invention are available as commercial products. Examples include Gambro's HCO^{®} 1100 and Theralite^{®} dialyzers.

The dialyzer in the apparatus of the present application is usually operated in hemodialysis (HD) mode, but it is also possible to use hemodiafiltration (HDF) or hemofiltration (HF) mode.

The apparatus of the present application comprises a device configured for on-line measurement of the myoglobin level in the dialysate leaving the dialyzer. In one embodiment, the myoglobin level in the dialysate is measured using optical spectroscopy. The dialysate is irradiated with light having wavelengths in the range of from 350 to 430 nm and the light passing through the dialysate is detected. In one embodiment, light in the wavelength range of from 390 to 420 nm is used. The light may be polychromatic or monochromatic. In one embodiment, a light-emitting diode (LED) is used as the light source. LEDs emitting light in this wavelength range are commercially available, for instance, UV LEDs (380 nm), "black light" LEDs (wavelength 400-410 nm), or violet LEDs (wavelength 420 nm). A photosensor (photodetector) conveniently is used to measure the light that has passed through the dialysate. Examples of suitable photosensors are photodiodes, phototransistors, and photo-resistors (Light Dependent Resistors, LDRs).

In one embodiment of the apparatus of the present application, the device configured for on-line measurement of the myoglobin level in the effluent dialysate comprises a flow-through measuring cell. The light source and the photosensor are arranged on opposing sides of the measuring cell, so that the light emitted from the light source passes through the dialysate flowing through the measuring cell and is detected by the photosensor. In another embodiment, the light source and the photosensor are integrated into the jaws of a clip or clamp which is configured to be mounted on transparent tubing conveying the effluent dialysate. In still another embodiment, the light source and the photosensor are integrated into a sleeve mounted on transparent tubing conveying the effluent dialysate. In still another embodiment, the light source and the photosensor form part of a mount on the dialysis monitor configured to receive transparent tubing conveying the effluent dialysate.

In one embodiment, the apparatus comprises
iii) a device configured to display the myoglobin level measured on-line.

When the myoglobin level in the dialysate is measured using optical spectroscopy, the output signal of the photosensor, which is an indicator of the myoglobin level, may be displayed, for instance, on a gauge, a meter, a display, or a recording device. In one embodiment, the progression of the output signal with time is recorded and displayed.

In another embodiment, the apparatus comprises
iii) a device operable to determine the change with time of the myoglobin level measured; and
iv) a device configured to display the change with time of the myoglobin level determined.

The values measured are processed to determine the change with time of the myoglobin level in the dialysate. An example of a suitable device for this purpose is a differentiating circuit which determines the first derivative of the signal corresponding to the measured level. Alternatively, the derivative can be determined from the measured values using a suitable computer program. The result may be displayed, for instance, on a gauge, a meter, a numerical display, or a computer monitor.

In still another embodiment, the apparatus comprises
iii) a device operable to determine the change with time of the myoglobin level measured;
iv) a device operable to compare the absolute value of the change with time of the myoglobin level determined to a predefined threshold; and
v) a device operable to provide an output signal when the absolute value of the change with time is equal to or smaller than the predefined threshold.

The absolute value of the change with time of the myoglobin level is periodically or continuously compared to a predefined threshold. An example of a suitable device for this purpose is a comparator circuit. Alternatively, a suitable computer program can perform this operation. When the absolute value of the change with time of the myoglobin level is smaller than the predefined threshold, an output signal is generated. This signal indicates that the treatment session can be terminated and may be used to inform the medical personnel, to trigger an alarm or even control the dialysis monitor to end the dialysis session. The threshold value is selected so that an output signal is provided when there is little or no change in the myoglobin level in the dialysate. In one embodiment, the threshold value is zero. The apparatus of the invention allows for automatic determination of the optimum duration of a rhabdomyolysis treatment. It helps to avoid excessive length of an individual treatment session, and at the same time ensures that a treatment session is not terminated prematurely, i.e. before a constant myoglobin level has been reached in the patient's blood.

It will be understood that the features mentioned above and those described hereinafter can be used not only in the combination specified but also in other combinations or on their own, without departing from the scope of the present invention.

The present invention will now be described in more detail in the examples below. It is to be understood that the examples are not intended to limit the scope of the present invention and are merely an illustration of a preferred embodiment of the invention.

### Example

2.5 L heparinized bovine plasma having a protein content of 61 g/L and comprising 200 mg/L myoglobin from equine heart was circulated through the blood side of a high cut-off dialyzer having a surface area of A=2.1 m² (Theralite^{®}, Gambro) at a flow rate of Q_{B}=200 mL/min. Acetate dialysis fluid was pumped through the dialysate side of the filter at a flow rate of Q_{D}=500 mL/min. The ultrafiltration rate was set to UF=0 mL/min.

Samples of dialysate and plasma were periodically taken and the absorbance of the samples at 410 nm wavelength was measured. The results are shown in Table 1.

**Table 1 Myoglobin level over time in dialysate and plasma**

| | Absorbance at 410 nm | |
|---|---|---|
| t [min] | Dialysate | Plasma Pool |
| 0 | 0.191 | 1.083 |
| 2 | 0.236 | - |
| 4 | 0.220 | - |
| 6 | 0.202 | - |
| 8 | 0.182 | - |
| 10 | 0.169 | - |
| 15 | 0.131 | - |
| 20 | 0.109 | - |
| 25 | 0.088 | - |
| 30 | 0.168 | 0.565 |
| 40 | 0.044 | - |
| 50 | 0.025 | - |
| 60 | 0.016 | 0.343 |
| 90 | 0.003 | 0.237 |
| 120 | 0.002 | 0.168 |
| 150 | 0.000 | - |
| 180 | 0.000 | 0.149 |

## Claims

1. An apparatus comprising
(i) a dialyzer comprising a blood compartment and a dialysate compartment, said compartments being separated from each other by a membrane which *in vivo* allows passage of myoglobin; the apparatus being **characterized by**
(ii) a device configured for on-line measurement of the myoglobin level in the dialysate leaving the dialyzer.

2. The apparatus of claim 1, further comprising iii) a device configured to display the myoglobin level measured on-line.

3. The apparatus of claim 1, further comprising
iii) a device operable to determine the change with time of the myoglobin level measured; and
iv) a device configured to display the change with time of the myoglobin level determined.

4. The apparatus of claim 1, further comprising
iii) a device operable to determine the change with time of the myoglobin level measured;
iv) a device operable to compare the absolute value of the change with time of the myoglobin level determined to a predefined threshold; and
v) a device operable to provide an output signal when the absolute value of the change with time of the myoglobin level determined is equal to or smaller than the predefined threshold.

5. The apparatus of any one of claims 1 to 4, wherein the dialyzer shows a sieving coefficient for myoglobin, determined in accordance with DIN EN 1283, of more than 0.5.

6. The apparatus of any one of claim 5, wherein the sieving coefficient for myoglobin, determined in accordance with DIN EN 1283, is at least 0.9.

7. The apparatus of any one of claims 1 to 6, wherein the dialyzer shows a sieving coefficient for albumin, determined in accordance with DIN EN 1283, of not more than 0.2.

8. The apparatus of any one of claim 7, wherein the sieving coefficient for albumin, determined in accordance with DIN EN 1283, is 0.1 or lower.

9. The apparatus of any one of claims 1 to 8, wherein the dialyzer is a capillary dialyzer.

10. The apparatus of any one of claims 1 to 9, wherein the device configured for on-line measurement of the myoglobin level in the dialysate leaving the dialyzer is configured to measure the myoglobin level in the dialysate using optical spectroscopy.

11. The apparatus of claim 10, wherein the device configured for on-line measurement of the myoglobin level in the dialysate leaving the dialyzer comprises a light-emitting diode configured to emit light in the wavelength range of from 390 to 420 nm.

12. The apparatus of claim 11, wherein the device configured for on-line measurement of the myoglobin level in the dialysate leaving the dialyzer comprises a photosensor configured to detect light in the wavelength range of from 390 to 420 nm.

13. The apparatus of claim 10, wherein the device configured for on-line measurement of the myoglobin level in the dialysate leaving the dialyzer comprises a light-emitting diode configured to emit light in the wavelength range of from 400 to 410 nm.

14. The apparatus of claim 13, wherein the device configured for on-line measurement of the myoglobin level in the dialysate leaving the dialyzer comprises a photosensor configured to detect light in the wavelength range of from 400 to 410 nm.

## Patentansprüche

1. Vorrichtung umfassend
(i) einen Dialysator mit einem Blutraum und einem Dialysatraum, wobei die Räume durch eine Membran voneinander getrennt sind, die *in vivo* den Durchtritt von Myoglobin zulässt; wobei die Vorrichtung **gekennzeichnet ist durch**
(ii) ein Gerät, das für die online-Messung des Myoglobinspiegels in dem den Dialysator verlassenden Dialysat eingerichtet ist.

2. Vorrichtung gemäß Anspruch 1, ferner umfassend iii) ein Gerät, das für die Anzeige des online gemessenen Myoglobinspiegels eingerichtet ist.

3. Vorrichtung gemäß Anspruch 1, ferner umfassend
iii) ein Gerät, das für die Ermittlung der zeitlichen Veränderung des gemessenen Myoglobinspiegels einsetzbar ist; und
iv) ein Gerät, das für die Anzeige der ermittelten zeitlichen Veränderung des Myoglobinspiegels eingerichtet ist.

4. Vorrichtung gemäß Anspruch 1, ferner umfassend
iii) ein Gerät, das für die Ermittlung der zeitlichen Veränderung des gemessenen Myoglobinspiegels einsetzbar ist;
iv) ein Gerät, das für den Vergleich des Absolutwerts der ermittelten zeitlichen Veränderung des gemessenen Myoglobinspiegels mit einem vorgegebenen Schwellenwert einsetzbar ist; und
v) ein Gerät, das einsetzbar ist, ein Ausgangssignal bereitzustellen, wenn der Absolutwert der ermittelten zeitlichen Veränderung des gemessenen Myoglobinspiegels gleich dem vorgegebenen Schwellenwert oder kleiner als dieser ist.

5. Vorrichtung gemäß einem der Ansprüche 1 bis 4, worin der Dialysator einen Siebkoeffizienten für Myoglobin aufweist, bestimmt gemäß DIN EN 1283, der größer als 0.5 ist.

6. Vorrichtung gemäß Anspruch 5, worin der Siebkoeffizient für Myoglobin, bestimmt gemäß DIN EN 1283, mindestens 0.9 beträgt.

7. Vorrichtung gemäß einem der Ansprüche 1 bis 6, worin der Dialysator einen Siebkoeffizienten für Albumin aufweist, bestimmt gemäß DIN EN 1283, der nicht größer ist als 0.2.

8. Vorrichtung gemäß Anspruch 7, worin der Siebkoeffizient für Albumin, bestimmt gemäß DIN EN 1283, 0.1 oder weniger beträgt.

9. Vorrichtung gemäß einem der Ansprüche 1 bis 8, worin der Dialysator ein Hohlfaserdialysator ist.

10. Vorrichtung gemäß einem der Ansprüche 1 bis 9, worin das Gerät, das für die online-Messung des Myoglobinspiegels in dem den Dialysator verlassenden Dialysat eingerichtet ist, dafür eingerichtet ist, den Myoglobinspiegel im Dialysat mittels optischer Spektroskopie zu messen.

11. Vorrichtung gemäß Anspruch 10, worin das Gerät, das für die online-Messung des Myoglobinspiegels in dem den Dialysator verlassenden Dialysat eingerichtet ist, eine Licht emittierende Diode umfasst, die dafür eingerichtet ist, Licht im Wellenlängenbereich von 390 bis 420 nm zu emittieren.

12. Vorrichtung gemäß Anspruch 11, worin das Gerät, das für die online-Messung des Myoglobinspiegels in dem den Dialysator verlassenden Dialysat eingerichtet ist, einen Fotosensor umfasst, der dafür eingerichtet ist, Licht im Wellenlängenbereich von 390 bis 420 nm zu detektieren.

13. Vorrichtung gemäß Anspruch 10, worin das Gerät, das für die online-Messung des Myoglobinspiegels in dem den Dialysator verlassenden Dialysat eingerichtet ist, eine Licht emittierende Diode umfasst, die dafür eingerichtet ist, Licht im Wellenlängenbereich von 400 bis 410 nm zu emittieren.

14. Vorrichtung gemäß Anspruch 13, worin das Gerät, das für die online-Messung des Myoglobinspiegels in dem den Dialysator verlassenden Dialysat eingerichtet ist, einen Fotosensor umfasst, der dafür eingerichtet ist, Licht im Wellenlängenbereich von 400 bis 410 nm zu detektieren.

## Revendications

1. Appareil comprenant
i) un dialyseur comprenant un compartiment pour le sang et un compartiment pour le produit de dialyse, lesdits compartiments étant séparés l'un de l'autre par une membrane qui permet in vivo le passage de myoglobine ;
l'appareil étant **caractérisé par**
ii) un dispositif configuré pour une mesure en ligne du niveau de myoglobine dans le produit de dialyse quittant le dialyseur.

2. Appareil selon la revendication 1, comprenant en outre
iii) un dispositif configuré pour afficher le niveau de myoglobine mesuré en ligne.

3. Appareil selon la revendication 1, comprenant en outre
iii) un dispositif dont la fonction est de déterminer le changement au cours du temps du niveau de myoglobine mesuré ; et
iv) un dispositif configuré pour afficher le changement au cours du temps du niveau de myoglobine déterminé.

4. Appareil selon la revendication 1, comprenant en outre
iii) un dispositif dont la fonction est de déterminer le changement au cours du temps du niveau de myoglobine mesuré ;
iv) un dispositif dont la fonction est de comparer la valeur absolue du changement au cours du temps du niveau de myoglobine déterminé à un seuil prédéfini ; et
v) un dispositif dont la fonction est de fournir un signal de sortie quand la valeur absolue du changement au cours du temps du niveau de myoglobine déterminé est égale ou inférieure au seuil prédéfini.

5. Appareil selon l'une quelconque des revendications 1 à 4, dans lequel le dialyseur présente un coefficient de criblage pour la myoglobine, déterminé en accord avec la norme DIN EN 1283, supérieur à 0,5.

6. Appareil selon la revendication 5, dans lequel le coefficient de criblage pour la myoglobine, déterminé en accord avec la norme DIN EN 1283, est d'au moins 0,9.

7. Appareil selon l'une quelconque des revendications 1 à 6, dans lequel le dialyseur présente un coefficient de criblage pour l'albumine, déterminé en accord avec la norme DIN EN 1283, qui ne dépasse pas 0,2.

8. Appareil selon la revendication 7, dans lequel le coefficient de criblage pour l'albumine, déterminé en accord avec la norme DIN EN 1283 est de 0,1 ou moins.

9. Appareil selon l'une quelconque des revendications 1 à 8, dans lequel le dialyseur est un dialyseur capillaire.

10. Appareil selon l'une quelconque des revendications 1 à 9, dans lequel le dispositif configuré pour la mesure en ligne du niveau de myoglobine dans le produit de dialyse quittant le dialyseur est configuré pour mesurer le niveau de myoglobine dans le produit de dialyse en utilisant la spectroscopie optique.

11. Appareil selon la revendication 10, dans lequel le dispositif configuré pour la mesure en ligne du niveau de myoglobine dans le produit de dialyse quittant le dialyseur comprend une diode électroluminescente configurée pour émettre de la lumière dans la plage de longueurs d'ondes de 390 à 420 nm.

12. Appareil selon la revendication 11, dans lequel le dispositif configuré pour la mesure en ligne du niveau de myoglobine dans le produit de dialyse quittant le dialyseur comprend un photodétecteur configuré pour détecter la lumière dans la plage de longueurs d'ondes de 390 à 420 nm.

13. Appareil selon la revendication 10, dans lequel le dispositif configuré pour la mesure en ligne du niveau de myoglobine dans le produit de dialyse quittant le dialyseur comprend une diode électroluminescente configurée pour émettre de la lumière dans la plage de longueurs d'ondes de 400 à 410 nm.

14. Appareil selon la revendication 13, dans lequel le dispositif configuré pour la mesure en ligne du niveau de myoglobine dans le produit de dialyse quittant le dialyseur comprend un photodétecteur configuré pour détecter la lumière dans la plage de longueurs d'ondes de 400 à 410 nm.
